Europäisches Patentamt

European Patent Office

Office européen des brevets

(11) Veröffentlichungsnummer: **0 265 852**
**A2**

## (12) EUROPÄISCHE PATENTANMELDUNG

(21) Anmeldenummer: 87115528.9

(22) Anmeldetag: 23.10.87

(51) Int. Cl.4: **C12N 15/00** , C12N 9/10 , C12P 13/08

Der (Die) Mikroorganismus (Mikroorganismen) ist (sind) bei American Type Culture Collection unter der (den) Nummer(n) 11303 hinterlegt worden.

(30) Priorität: **29.10.86 DE 3636722**

(43) Veröffentlichungstag der Anmeldung:
**04.05.88 Patentblatt 88/18**

(84) Benannte Vertragsstaaten:
**AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Marquardt, Rüdiger, Dr.**
**Günthersburgallee 69**
**D-6000 Frankfurt am Main(DE)**
Erfinder: **Then, Johann, Dr.**
**Sulzbacher Weg 2**
**D-6238 Hofheim am Taunus(DE)**
Erfinder: **Deger, Hans-Matthias, Dr.**
**Am Rheingauer Weg 8**
**D-6238 Hofheim am Taunus(DE)**
Erfinder: **Wöhner, Gerhard, Dr.**
**Flörsheimer Strasse 27**
**D-6093 Flörsheim am Main(DE)**
Erfinder: **Robinson, Martyn, Dr.**
**45, Prince Andrew Road**
**Maidenhead Berkshire, SL6 8QQ(GB)**
Erfinder: **Doherty, Andrew, Dr.**

**verstorben(GB)**

(54) **Klonierung und Verwendung des Transaminase-Gens ilvE.**

(57) Das ilvE-Gen aus dem Stamm E. coli ATCC 11303 eignet sich zur Erzielung einer Überproduktion der kodierten aliphatischen Transaminase und damit zur Herstellung der verzweigtkettigen Aminosäuren Leucin, Isoleucin und Valin aus den entsprechenden Ketovorstufen.

EP 0 265 852 A2

## Klonierung und Verwendung des Transaminase-Gens ilvE

Der letzte Schritt der de novo-Synthese von aliphatischen Aminosäuren besteht in der Aminierung der entsprechenden Ketovorstufen mit Hilfe einer Transaminase. Obwohl verschiedene Transaminasen in der Lage sind, Transaminierungsreaktionen durchzuführen, die zu aliphatischen Aminosäuren führen, wird diese Funktion in der Zelle hauptsächlich von der sogenannten aliphatischen Transaminase erfüllt. Die genetische Abkürzung für das Gen der aliphatischen Transaminase ist ilvE (Umbarger, Ann. Rev. Biochemistry 47 (1978), 533-606). Im Falle von E. coli K12 ist die Lage des Gens auf dem "Bakterienchromosom" genau bekannt, und das Genprodukt hat die E.C.-Nummer 2.6.1.42 erhalten (Bachmann et. al., Microbiological Reviews 44 (1980), 1-56).

In der Europäischen Patentanmeldung (EP-A) 0 116 860 wird die Isolierung des ilvE-Gens aus E. coli K12 sowie die Klonierung dieses Aminotransferase-Gens auf ein Multi-Copy-Plasmid beschrieben. Spezifische Angaben zur Aktivität des klonierten ilvE-Genprodukts werden jedoch nicht gemacht. In der EP-A 0 152 275 wird erwähnt, daß durch die Klonierung des ilvE-Gens eine Steigerung der Enzymaktivität erreicht werden kann. Angaben über eine Ausbeutesteigerung an aliphatischen Aminosäuren finden. sich jedoch nicht.

Es wurde nun überraschend gefunden, daß die Isolierung des in E. coli ATCC 11303 vorhandenen ilvE-Gens und dessen Klonierung auf ein Multi-Copy-Plasmid nach der Transformation des Ausgangsstamms mit diesem Plasmid in einer mindestens 3-bis 5-fachen Steigerung der Ausbeute an der jeweiligen Aminosäure resultiert.

Die Erfindung betrifft somit:

1. Ein replizierendes extra-chromosomales Element, welches das aus E. coli ATCC 11303 isolierte ilvE-Gen enthält.

2. Die Verwendung des unter 1) genannten extra-chromosomalen Elements zur Synthese der aliphatischen Aminotransferase.

3. Die Verwendung des· unter 1) genannten extra-chromosomalen Elements zur Überproduktion von. verzweigtkettigen Aminosäuren in Mikroorganismen, die dadurch gekennzeichnet ist, daß

a) das extra-chromosomale Element in einen Mikroorganismus eingebracht wird,

b) das ilvE-Gen in diesem Mikroorganismus exprimiert wird und eine aktive aliphatische Transaminase synthetisiert und

c) die Transaminase die Aminierung der entsprechenden Ketovorstufen bewirkt.

Die Erfindung wird in der folgenden Beschreibung detailliert erläutert bzw. in den Patentansprüchen definiert.

Außer dem Wildtyp E. coli ATCC 11303 können auch dessen Varianten und Mutanten verwendet werden. Beispielsweise kann auch ein nach bekannten Methoden mutierter Stamm [E. Adelberg et al., Biochem. Biophys. Res. Comm. 18, 788 (1965)], der auf Überproduktion von verzweigtkettigen Aminosäuren selektioniert wurde, eingesetzt werden. Die aliphatische Aminotransferase aus E. coli ATCC 11303, für die das ilvE-Gen codiert, synthesiert unter anderem Valin, Leucin und Isoleucin aus den jeweiligen Ketovorstufen, indem eine Aminogruppe aus Glutamat transferiert wird. Daneben können mit Hilfe der aliphatischen Transaminase auch Phenylalanin und Glutaminsäure synthetisiert werden. Neben dem Produkt des ilvE-Gens finden sich als Produkte anderer Gene weitere Transaminasen in E. coli-Zellen.

Beispielsweise katalysiert die aromatische Transaminase, das Produkt des tyrB-Gens, die Synthese von Phenylalanin, Tyrosin, Aspartat und Leucin und das Produkt des aspC-Gens die Synthese von Aspartat, Glutamat, Phenylalanin und Tyrosin. Allerdings zeigt jede der genannten Transaminasen auch eine - schwache Aktivität bei der Synthese von Aminosäuren, die eigentlich spezifischer einer der· beiden anderen Transaminasen zuzuordnen sind.

Um die Synthese von verzweigtkettigen Aminosäuren noch weiter zu erhöhen, wird das ilvE-Gen, das für die aliphatische Aminotransferase codiert, kloniert. Man erreicht dies durch Isolierung der DNA aus E. coli ATCC 11303. Nach partieller Verdauung der DNA werden die entstandenen Fragmente, die sich in einm Größenbereich von 20-30 kb bewegen, in ein Cosmid mit einem Replikon, das einen weiten Wirtsbereich vermittelt, ligiert und in die Köpfe des λ-Phagen verpackt. Bevorzugt wird das Cosmid pIMS 6026 verwendet. Das Cosmid pIMS 6026 leitet sich von dem Cosmid pLAFR 1 (ATCC 37167) dadurch ab, daß in die einzige EcoRI-Schnittstelle des Cosmids pLAFR 1 das handelsübliche EcoRI-Fragment (Pharmacia, Upsala, Schweden) kloniert wurde, auf dem das Kanamycin-Resistenzgen des Transposons Tn 903 liegt. Durch Verdauung mit BamHI und anschließende Religation kann der größte Teil des Kanamycin-Gens deletiert werden, so daß ein kurzes Stück DNA als Insertion zurückbleibt, in der eine BamHI-Schnittstelle von 2 EcoRI-Schnittstellen flankiert wird. Diese BamHI-Schnittstelle, die auf dem Cosmid pLAFR 1 nicht vorhanden ist, kann für Klonierungen verwendet werden. Durch Inkubation der verpackten Cosmide mit einer entsprechend vorbereiteten E. coli DG 30-Suspension werden die Cosmide in den Mikroorganismus eingeschleust. E. coli DG 30 hat eine Defizienz in den drei Transaminasen aspC, ilvE und tyrB. Der Stamm wächst dahere auf Vollmedium zwar problemlos an, muß aber bei Wachstum auf Minimalmedium mit verschiedenen Aminosäuren von außen versorgt werden, da er sie nicht selbst synthetisieren kann. Bei geeigneter Wahl des Mediums kann mit Hilfe des Stamms untersucht werden, ob eine von außen aufgenommene DNA den chromosomalen Defekt für eine bestimmte Transaminase komplementieren kann. Die Einschleusung des ilvE-Gens detektiert man durch Wachstum des E. coli DG 30 auf einem tyrosinfreien Minimalmedium. Auf diesem Medium können nur Klone anwachsen, die durch Aufnahme von aspC, tyrB oder ilvE enthaltender DNA, die ursprünglich aus dem Stamm E. coli ATCC 11303 stammt, ihren chromosomalen Defekt für die Synthese von Tyrosin komplementieren können. Die drei Transaminasen, die von den Genen aspC, tyrB und ilvE codiert werden, unterscheiden sich in ihrer Substratspezifität, wenngleich sie alle drei in der Lage sind, Tyrosin zu bilden.

Die aromatische Transaminase, die von dem Gen tyrB codiert wird, kann beispielsweise kein Isoleucin aus der Ketovorstufe bilden, wohl aber Leucin in guten Ausbeuten aus der entsprechenden Ketovorstufe synthetisieren. Die Transaminase, die von dem Gen aspC codiert wird, kann weder Isoleucin bilden, noch ist sie effizient in der Umsetzung zu Leucin. Das ilvE-Genprodukt bewirkt aber eine gute Ausbeute an Isoleucin.

Dementsprechend können die einzelnen Klone durch Wachstum auf einem Minimalmedium, das bis au eine für das jeweilige Gen charakteristische Aminosäure mit für den Soffwechsel essentiellen Aminosäuren supplementiert ist, hinsichtlich der enthaltenen Transaminase unterschieden werden. Auf diese Weise werden Klone des DG 30-Stammes, die das ilvE-Gen enthalten, heraus selektioniert.

Es muß nun überprüft werden, ob die Klone auch wirklich das ilvE-Gen besitzen. Hierzu muß die Plasmid-DNA aus den Klonen isoliert werden. Die Isolierung aus dem Stamm DG 30 gelingt jedoch nur mit Schwierigkeiten. Es kann zwar Plasmid-DNA erhalten werden, jedoch nur in geringen Ausbeuten. Nach Minilyse wird daher ein E. coli-Stamm mit den Cosmid-DNAs aus den interessanten Klonen tranformiert, aus der anschließend die eingeschleuste DNA in guten Ausbeuten reisoliert werden kann. E. coli DH1 (ATCC 33849) ist hierzu insbesondere geeignet.

In dem nächsten Schritt wird die reisolierte Cosmid DNA mit einem Vektor hoher Kopienzahl ligiert. Aus der Chromosomalen Genkarte des Stammes E. coli K12 ist bekannt, daß das ilvE-Gen auf einem sall-Smal-Fragment lokalisiert ist. Dieses Fragment enthält nur das Strukturgen von ilvE, nicht aber den natürlichen Promotor. Es besteht die Möglichkeit, daß dies auch bei E. coli ATCC 11303 der Fall ist. Aus diesem Grund wird als Vektor bevorzugt ein Multi-Copy-Plasmid eingesetzt, welches eine Klonierung des Sall-Smal-Fragments dergestalt erlaubt, daß die klonierte Sequenz unter die Kontrolle eines auf dem Plasmid vorhandenen Promotors gelangt. Insbesondere bevorzugt ist das in seiner Sequenz bekannte und handelsübliche pBR322.

Die Cosmid-DNA wird mit den Enzymen Sall und Smal, der Vektor mit den Restriktionsenzymen Sall und Pvull vollständig verdaut. Die beiden DNAs werden zusammengegeben, miteinander ligiert und kompetente Zellen eines Wirtsorganismus, in dem die Produktion verzweigtkettiger Aminosäuren erhöht werden soll, damit transformiert. Bevorzugt werden Enterobakterien eingesetzt, insbesondere E. coli, wobei E. coli ATCC 11303 sowie seine Mutanten und Varianten besonders bevorzugt sind. Resistente Kolonien werden über Ampicillin selektioniert, aus den entsprechenden Kolonien durch Minilyse Plasmid-DNA isoliert und durch vollständige Verdauung mit dem Restriktionsenzym Sall die Vektor-DNA auf Größenveränderung überprüft. Durch Restriktionsanalyse wird sichergestellt, daß alle in dem ursprünglichen DNA-Abschnitt enthaltenen Sall-Smal-Fragmente auf diese Art subkloniert worden sind. Klone, die jeweils eins dieser Sall-Smal-Fragemte enthalten, werden schließlich mittels eines literaturbekannten Tests (Duggan et al., Anal. Biochem. 51 (1973) 67-79) auf die Aktivität der aliphatischen Transaminase, also des Genprodukts von ilvE, geprüft. Auf diese Weise läßt sich eine Zunahme der ilvE-Aktivität um einen Faktor von mehr als 10 erreichen. Durch Agarose-Gelelektrophorese kann gezeigt werden, daß in dem Vektor des Wirtsstammes ein ca. 1 MD großes Fragment aus ATCC 11303 enthalten ist.

In den folgenden Beispielen wird die Erfindung im einzelnen beschrieben. Prozentangaben beziehen sich, wenn nicht anders angegeben, auf das Gewicht.

Beispiel 1

Isolierung und Verdauung des Cosmids pIMS 6026 aus E. coli

Für die Isolierung des Cosmids pIMS 6026 aus E. coli wurde entweder nach Humphreys et al. vorgegangen [Biochem. Biophys. Acta 383, 457-63 (1975)] oder eine alkalische Lyse nach Birnboim und Doly [Nucleic Acids Res. 7 : 151] im 10-fach größeren Maßstab durchgeführt. In jedem Fall wurde die Plasmid-DNA mindestens einmal durch CsCl/EtBr-Dichtegradientenzentrifugation gereinigt.

Das Cosmid pIMS 6026 wurde vollständig mit dem Restriktionsenzym BamHI verdaut, wobei nach den Angaben des Herstellers, New England Biolabs, verfahren wurde. Zur Überprüfung der Vollständigkeit dieser Verdauung wurde ein Aliquot des Restriktionsansatzes auf ein 0,8 %iges Agarosegel aufgetragen und der Elektrophorese unterworfen. Das Erscheinen nur einer Bande nach Anfärben mit Ethidiumbromid und Bestrahlung mit kurzwelligem UV-Licht (254nm) diente als Hinweis auf eine vollständige Verdauung. Von der verdauten Cosmid-DNA wurde das Restriktionsenzym durch Phenolisierung entfernt, die DNA mittels Ethanol gefällt, mit 70 %igem Ethanol gewaschen und nach Trocknen unter Vakuum in einem geeigneten Volumen an TE-Puffer(10 mM Tris; 1 mM EDTA, pH 8,0) aufgenommen. Wahlweise wurde noch eine Behandlung mit alkalischer Phosphatase nach den Angaben des Herstellers, Boehringer Mannheim, durchgeführt. Nach Zugabe von 1 µl an alkalischer Phosphatase (CIP) wurde 30 Minuten lang bei 37°C inkubiert, das Enzym durch Phenolisierung aus dem Reaktionsansatz entfernt und die DNA, wie oben beschrieben, gereinigt. Schließlich wurde sie in TE-Puffer resuspendiert.

Beispiel 2

Partielle Verdauung der DNA aus E. coli ATCC 11303

Die Isolierung der Gesamt-DNA aus E. coli ATCC 11303 wurde nach der Methode von Marmur in J. Mol. Biol. 53, 155-162, (1961) durchgeführt. Die isolierte Gesamt-DNA wurde mit dem Restriktionsenzym Sau3A partiell verdaut, so daß hauptsächlich Fragmente in einem Größenbereich von 20-30kb entstanden. Dazu wurde in Vorversuchen das hierfür optimale Verhältnis von DNA und Enzym sowie die optimale Einwirkdauer des Enzyms auf die DNA festgestellt. Die entsprechende Vorgehensweise ist in dem von der Firma BRL herausgegebenen Heft "focus", Vol. 7, Nr 2 (1985) auf Seite 3 beschrieben. Nach Ablauf der als optimal bestimmten Reaktionszeit wurde das Enzym durch Erhitzen auf 65°C über einen Zeitraum von 10 Minuten zerstört und die Bildung von DNA-Fragmenten im gewünschten Größenbereich durch Agarose-Gelelektrophorese mit geeigneten DNA-Markern, z.B. mit EcoRI-verdauter DNA des Phagen λ, überprüft.

Beispiel 3

Ligation der Restriktionsansätze

Mit Sau3A partiell verdaute Gesamt-DNA aus E. coli ATCC 11303 wurde mit pIMS 6026 Cosmid-DNA, die mit BamHI vollständig gespalten und mit alkalischer Phosphatase behandelt worden war, in einem molaren Verhältnis von etwa 1 : 5 zusammengegeben. Die resultierende Mischung wurde mit einem mehrfach konzentrierten Puffer nach den Angaben von New England Biolabs so versetzt, daß eine für das Enzym T4-DNA-Ligase optimale Ionenkonzentration resultierte, und mit 1 µl des Enzyms mindestens 14 Stunden lang bei 16°C inkubiert Das Gesamtvolumen des Ansatzes betrug dabei 50 µl mit einer Gesamt-DNA-Konzentration von 20 µg/ml.

Beispiel 4

Verpackung in λ-Phagen

Nach erfolgter Ligase-Reaktion wurde nach Beispiel 3 erhaltene DNA in vitro in die Köpfe von λ-Phagen verpackt. Die dafür notwendigen Extrakte aus zwei verschiedenen Bakterienstämmen können nach Hohn, B., in Wu, R., editor: Recombinant DNA, Methods in Enzymology, Vol. 68, Academic Press, New York, S.299-309 (1979) gewonnen werden oder von Boehringer Manheim oder Amersham Buchler, Braunschweig, bezogen werden.

3µl der nach Beispiel 3 erhaltenen Mischung wurden mit unmittelbar zuvor aufgetauten Bakterien-Extrakten der Firma Amersham unter Eiskühlung gründlich vermischt. Die Mischung wurde 30-60 Minuten bei 20°C inkubiert und anschließend 200µl SM-Puffer (100 nM NaCl, 10 mM MgSO₄, 50 mM Tris-HCl (pH 7,5), 0,01 % Gelatine) zugegeben. Diese Mischung wurde entweder direkt in eine Transduktions-Reaktion eingesetzt oder nach Zugabe von 10 µl Chloroform bis zur späteren Verwendung bei 4°C aufbewahrt.

Beispiel 5

Transduktion von E. coli DG 30

5 ml L-Broth, bestehend aus 1 % Bacto-Trypton, 0,5 % Hefeextrakt und 0,5 % NaCl, wurden 0,4 % Maltose zugesetzt und mit 50 µl einer Flüssigkultur von E. coli DG 30 in der stationären Wachstumsphase beimpft. Es wurde 12 Stunden bei 37°C inkubiert, bis die frühre stationäre Phase erreicht war. Die Bakterien wurden abzentrifugiert und vorsichtig in 2,5 ml einer wäßrigen Lösung, die 10 mmolar an MgCl₂ war, resuspendiert. 10 µl der Mischung gemäß Beispiel 4 wurden mit 20 µl der konzentierten Bakteriensuspension versetzt und 50 Minuten bei Raumtemperatur inkubiert.

Anschließend wurde 200 µl L-Broth hinzugegeben und 1 Stunde bei 37°C inkubiert, wobei die Mischung gelegentlich geschüttelt wurde.

Jeweils 50 µl des Ansatzes wurden auf L-Broth-Agar, der 20 µg/ml Tetracyclin enthielt, ausplattiert. Die Platten wurden mindestens 12 Stunden bei 37°C bebrütet. Bei der beschriebenen Vorgehensweise konnten aus einem Ansatz durchschnittlich 1000 Kolonien erhalten werden.

Beispiel 6

Selektionierung von E. coli DG 30 mit einem aspC-oder ilvE-oder tyrB-Gen.

Rund 800 Kolonien, die nach Transduktion von E. coli DG 30 nach dem beschriebenen Verfahren auf L-Broth-Agar, der 20 µg/ml Tetracyclin enthielt, erhalten worden waren, wurden auf Minimalager "gepickt". Der Minimalager bestand aus M9-Medium mit Glukose (Miller, Experiments in Molecular Genetics, Cold Spring Harbor, 1972), das durch die Aminosäuren Isoleucin, Leucin, Valin, Asparaginsäure und Phenylalanin supplementiert worden war. Die Aminosäure Tyrosin, die der Stamm DG 30 gleichfalls nicht mehr synthetisieren kann, wurde dem Medium jedoch nicht zugegeben. Von den 800 "gepickten" Kolonien konnten 7 auf dem Minimalmedium anwachsen.

Zur Unterscheidung der drei möglichen Gene aspC, ilvE und tyrB in E. coli DG 30 wurden diese 7 Kolonien wiederum auf oben genanntes Minimalmedium "gepickt", das mit den aufgeführten Aminosäuren supplementiert war, bis auf jeweils eine, für die eine der Transaminasen, die von einem der Gene codiert wird, Substratspezifität zeigt. Das Ergebnis ist in der nachstehenden Tabelle aufgeführt:

| Klon | Minimalmedium, supplementiert bis auf | | | | vermutliches Gen |
|------|------|------|------|------|------|
| | Asp | Leu | Ile | tyr | |
| 1 | + | + | - | + | tyrB |
| 2 | + | + | - | + | tyrB |
| 3 | - | +- | + | +- | ilvE |
| 4 | - | +- | + | +- | ilvE |
| 5 | + | + | - | + | tyrB |
| 6 | + | + | - | + | tyrB |
| 7 | - | +- | + | +- | ilvE |

+  = gutes Wachstum

+- = schlechtes Wachstum

-  = kein Wachstum

Beispiel 7

Lokalisierung des ilvE-Gens

Durch Minilyse nach Maniatis et al., Cold Spring Harbor, Seiten 366-370 (1982), wurde Cosmid-DNA der Klone 1 bis 7, die gemäß Beispiel 6 erhalten wurden, gewonnen. Anschließend wurde diese Cosmid-DNA in E. coli DH1 (ATCC 33849) eingeschleust, woraus sie in guten Ausbeuten wieder reisoliert werden konnte. Es wurde Plasmid-DNA, die ursprünglich aus dem Klon 3 von E. coli DG 30 (siehe Beispiel 6) gewonnen wurde, aus dem mit dieser DNA transformierten Stamm E. coli DH 1 isoliert und mit den Restriktionsenzymen SalI und SmaI, den Angaben des Herstellers, New England Biolabs, folgend, vollständig verdaut. Gleichfalls vollständig mit den Enzymen SalI, PvuII und AvaI verdaut wurde der Vektor pBR322. Die Verdauung mit AvaI soll ein Zurückligieren des aus pBR322 stammenden SalI-SmaI-Fragments in den Vektor verhindern. Die beiden DNAs wurden zusammengegeben, nach der in Beispiel 4 bereits beschriebenen Art und Weise miteinander ligiert und kompetente Zellen des Stammes E. coli ATCC 11303 mit einem Aliquot des Ligase-Ansatzes, z.B. 10 µl, transformiert. Resistente Kolonien wurden auf L-Broth-Platten, die 50 µg/ml Ampicillin enthielten, selektioniert, durch Minilyse Plasmid-DNA isoliert und durch vollständige Verdauung mit dem Restriktionsenzym SalI die Vektor-DNA auf Größenveränderung überprüft.

Beispisel 8

Überprüfung der Transaminase-Aktivität

Die gemäß Beispiel 7 erhaltenen Klone wurden mittels des genannten Tests auf die Aktivität der aliphatischen Transaminase, also des Genprodukts von ilvE, getestet. Als Vergleich diente der nicht transformierte Ausgangsstamm E. coli ATCC 11303. Dabei konnte in einem Fall eine deutliche Zunahme an ilvE-Aktivität, und zwar um den Faktor größer als 10, gegenüber dem Ausgangsstamm E. coli ATCC 11303, gemessen werden.

Durch Agarose-Gelelektrophorese unter Verwendung geeigneter Marker konnte gezeigt werden, daß in dem Stamm, der erhöhte ilvE-Genaktivität aufwies, ein pBR322-Vektor enthalten war, der ein rund 1 MD großes Fragment aus ATCC 11303 eingebaut enthielt. Wurde mit der isolierten Plasmid-DNA erneut der plasmidlose Stamm E. coli ATCC 11303 tranformiert so konnte in jedem Fall eine Zunahme der ilvE-Genaktivität um einen Faktor von größer als 10 beobachtet werden.

Beispiel 9

Temperaturinduzierbare Expression des ilvE-Gens

Das als SalI-Fragment von rund 1 MD klonierte DNA-Fragment aus E. coli ATCC 11303 trägt das ilvE-Gen ohne den natürlichen Promotor. Vielmehr erfolgt die Expression des Gens nach Klonierung in SalI-PvuII-verdautes pBR322 durch Transkription ausgehend vom Tetracyclin-Promotor aus pBR322 und anschließender Translation der zu ilvE korrespondierenden mRNA. Der Tetracyclin-Promotor aus pBR322 ist zwar relativ stark und führt zu guten Proteinausbeuten, kann jedoch nicht durch Temperaturveränderung reguliert werden. Um die Expression von ilvE temperaturregulierbar zu machen, wird das den Tetracyclin-Promotor tragende SalI-EcoRI-Fragment am 5'-Ende des ilvE-Gens durch ein SalI-EcoRI-Fragment ausgetauscht, auf dem der $P_R$-Promotor des Phagen λ sowie das temperatur-sensitive Repressorgen cl857 lokalisiert sind. Als Quelle für dieses Fragment dient der Vektor pCQV2 (Queen, J. Molec. and Applied Genetics 2 (1983) 1-10). Der Vektor pCQV2 wird mit den Restriktionsenzymen EcoRI und SalI vollstandig verdaut. Gleichfalls vollständig mit diesen Enzymen verdaut wird der Vektor, der nach Beispiel 8 gewonnen wird. Hierzu werden die jeweiligen Enzyme gleichzeitig der DNA zugesetzt und der Ansatz in REB-Puffer [50 mM NaCl, 10 mM Tris-HCl, pH 7,5; 6 mM 2-Mercaptoethanol, 6 mM MgCl2, 100 μg/ml Rinderserumalbumin, 0,01 % nichtionisches Tensid (®Triton-X-100)] für eine Stunde bei 37°C inkubiert. Das Gesamtvolumen der Ansätze beträgt 50 μl bei einer DNA-Konzentration von 20 μg/ml. Durch Agarose-Gelelektrophorese eines 5 μl Aliquots der beiden Ansätze wird die Vollstäandigkeit der Verdauungen übferprüft. Die beiden Ansätze werden 10 Minuten lang auf 65°C erhitzt, die Restriktionsenzyme durch Phenolisierung entfernt und die geschnittene Plasmid-DNA durch Ethanol-Fällung sowie anschließendes Waschen mit 70 %igem Ethanol gereinigt. Nach Trocknen unter Vakuum werden die jeweiligen DNAs in 50 μl TE-Puffer resuspendiert und 10 μl der beiden Ansätze zusammenpipettiert. Die Mischung wird auf das vom Hersteller (New England Biolabs) als optimal für das Enzym T4-DNA-Ligase empfohlene Milieu eingestellt und mit 10 Units des Enzyms T4-DNA-Ligase versetzt. Der Ansatz wird 16 Stunden lang bei 16°C inkubiert und anschließend kompetente Zellen des E. coli-Stammes HB 101 mit einem 10 μl-Aliquot des Ligase-Ansatzes transformiert Ampicillin-resistente Kolonien werden selektioniert und nach Minilyse Plasmid-DNA aus diesen Klonen mit den Restriktionsenzymen SalI und EcoRI einer Doppelverdauung unterzogen. Durch Agarosegelelektrophorese können Klone identifiziert werden, die Plasmide mit dem erwarteten Fragmentmuster enthalten.

Zwei der so identifizierten Klone werden in LB-Medium (Miller, a.a.O.) angezüchtet, wobei die Inkubationstemperatur 30°C bzw. 37°C beträgt. Wird die Enzymaktivität des ilvE-Genprodukts aus bei 37°C angezüchteten Bakterien untersucht, so ergibt sich ein Wert, der um einen Faktor von 5-10 über dem eines plasmidlosen Vergleichsstammes liegt. Wird die Enzymaktivität des bei 30°C angezüchteten Klons untersucht, so sind die gemessenen Werte praktisch mit denen eines plasmidlosen Vergleichstammes identisch. Werden die Bakterien dagegen bei 30° angezüchtet, die Temperatur der Kultur sodann durch Schütteln in einem 65°C warmen Wasserbad auf 45°C erhöht und die Kultur anschließend bei 37°C für 2 Stunden weiterinkubiert, so können mindestens 10-fach erhöhte Enzymaktivitäten gemessen werden.

**Ansprüche**

1. Ein replizierendes extra-chromosomales Element, welches das aus E. coli ATCC 11303 isolierte ilvE-Gen enthält.

2. Das extra-chromosomale Element nach Anspruch 1, dadurch gekennzeichnet, daß es ein Multi-Copy-Plasmid ist.

3. Das extra-chromosomale Element nach Anspruch 2, dadurch gekennzeichnet, daß sich das Multi-Copy-Plasmid von pBR322 ableitet.

Verwendung des extra-chromosomalen Elements nach einem oder mehreren der Ansprüche 1 bis 3 zur Synthese der aliphatischen Transaminase.

5. Verwendung des extra-chromosomalen Elements nach einem oder mehreren der Ansprüche 1 bis 3 zur Überproduktion von verzweigtkettigen Aminosäuren in Mikroorganismen, dadurch gekennzeichnet, daß

a) das extra-chromosomale Element in einen Mikroorganismus eingebracht wird,

b) das ilvE-Gen in dem Mikroorganismus exprimiert wird und eine aktive aliphatische Transaminase synthetisiert und

c) die Transaminase die Aminierung der entsprechenden Ketoverstufen bewirkt.

7

6. Verwendung nach Anspruch 5, dadurch gekennzeichnet, daß der Mikroorganismus ein Entero-Bakterium ist.

7. Verwendung nach Anspruch 5, dadurch gekennzeichnet, daß der Mikroorganismus E. coli ist.

8. Verwendung nach Anspruch 6, dadurch gekennzeichnet, daß der Mikroorganismus E. coli ATCC 11303 sowie dessen Varianten und Mutanten ist.

9. E. coli ATCC 11303 sowie dessen Varianten und Mutanten, transformiert mit dem extra-chromosomalen Element nach einem oder mehreren der Ansprüche 1 bis 3.

10. ilvE-Gen, erhältlich aus E. coli ATCC 11303 sowie dessen Varianten und Mutanten.

11. Genstruktur, bestehend aus dem ilvE-Gen nach Anspruch 10, unter der temperaturregulierbaren Kontrolle des $\lambda$-$P_R$-Promotors.

Patentansprüche fur die folgenden Vertragsstaaten

1. Verfahren zur Herstellung eines ein ilvE-Gen enthaltenden replizierenden extrachromosomalen Elements, dadurch gekennzeichnet, daß das ilvE-Gen aus E. coli ATCC 11 303 isoliert und in ein Plasmid cloniert wird.

2. Verfahren nach Anspruch 1, dadurch gekennzeichnet, daß als Plasmid ein Multi-Copy-Plasmid eingesetzt wird.

3. Verfahren nach Anspruch 2, dadurch gekennzeichnet, daß als Plasmid pBR 322 eingesetzt wird.

4. Verfahren zur Überproduktion von verzweigtkettigen Aminosäuren in Mikroorganismen, dadurch gekennzeichnet, daß

a) das extrachromosomale Element, erhältlich nach Anspruch 1, in einen Mikroorganismus eingebracht wird,

b) das ilvE-Gen in dem Mikroorganismus exprimiert wird und eine aktive aliphatische Transaminase synthetisiert und

c) die Transaminase die Aminierung der entsprechenden Ketovorstufen bewirkt.

5. Verfahren nach Anspruch 4, dadurch gekennzeichnet, daß der Mikroorganismus ein Enterobakterium ist.

6. Verfahren nach Anspruch 5, dadurch gekennzeichnet, daß der Mikroorganismus E. coli ist.

7. Verfahren nach Anspruch 6, dadurch gekennzeichnet, daß der Mikroorganismus E. coli ATCC 11303 sowie dessen Varianten und Mutanten ist.